# EUROPEAN PATENT APPLICATION

(11) **EP 4 751 635 A1**
(43) Date of publication of application: **03.06.2026**
(21) Application number: 24383301.9
(22) Date of filing: 29.11.2024
(51) Int. Cl.: A61B 1/00, A61B 1/07

(54) **SURGICAL DEVICE AND METHOD**

(71) Applicant: Palau i Bertran, Nil, 08013 Barcelona (ES); Palau González, Jordi, 08013 Barcelona (ES)
(72) Inventor: Palau i Bertran, Nil, 08013 Barcelona (ES); Palau González, Jordi, 08010 Barcelona (ES); Bertran Serra, Immaculada, 08010 Barcelona (ES)

(57) **Abstract**

The present application relates to a surgical device that comprises a light connector and an optical device. The light connector is configured at least to receive visible light such that the surgical device illuminates an interior portion of an anatomic body. The optical device includes an optical body, for example, a lens body. An image sensor is further provided in optical communication with the optical body. In this way, the image sensor is configured to acquire a captured image of the interior portion of the anatomic body illuminated by the visible light. The surgical device further comprises a circuit board, a controller, and a transceiver, wherein the controller is attached to the circuit board. The image sensor and the transceiver are electrically connected to the controller, and wherein the controller is configured at least to transmit image data derived at least partially from raw image data generated by the image sensor to one or more user devices in a wireless manner through the transceiver. The surgical device is particularly a wireless surgical device such as a cable-free arthroscopy device. Furthermore, a wireless surgical system is provided. The wireless surgical system comprises the surgical device and the one or more user devices. A method for operating the wireless surgical system is also provided.

## Description

### TECHNICAL FIELD

The present disclosure relates to a surgical device, a wireless surgical system comprising said surgical device, and a related method. In particular, the surgical device may be a wireless surgical device, such as a cable-free arthroscopy device, configured to wirelessly transmit at least image data to one or more user devices.

### BACKGROUND

Nowadays, surgery can be performed in two main ways. The first one, known as "open surgery", involves making an incision to expose the affected body part. Since a wide incision is required to enable the surgeon to directly access the affected area, this method comes with a high risk of damaging nearby structures. The procedure being highly invasive, it is inevitable for a patient to need a long recovery, and the likelihood of post operation complications, such as infections, remains high.

The second way of performing surgery is what is known as a "minimal-invasive surgery". This approach involves introducing an optical device into the patient's body through a small incision. This allows the surgeon to obtain a clear view of the problem and perform a second incision to insert the required tools for the surgery. Since an image assisted method only requires small incisions (0.5-1.5 cm), it is minimally invasive and has several benefits. Allowing minimal disruption of the body's tissues, this procedure considerably decreases post-surgery complications and can significantly speed up the recovery time. It is also worth mentioning that nowadays many of the minimal-invasive surgery can be converted to open surgery if complications arise or visibility is inadequate.

Some of the most common minimal-invasive surgeries are laparoscopic surgery, arthroscopy, or endoscopy.

A surgical system helps a surgeon to perform surgical operations. The surgical system typically comprises a surgical device and a surgical tower or user device. The surgical tower may comprise two or more consoles. Further, the surgical tower may comprise wheels, e.g., four wheels at the bottom portion of the surgical tower, to move it as required.

A surgical device may be, for example, an arthroscopy device. If so, the surgical tower may be an arthroscopic tower. Typically, the arthroscopy device employs cables or wires to be connected to the user device. The user device may be the surgical tower or one or more consoles to be used by a surgeon or surgical team.

The arthroscopy device of the prior art comprises an optical device, and an arthroscopy camera. The arthroscopy camera is configured to acquire a captured image of the interior portion of the patient, e.g., the human body. The prior art arthroscopy device is also connected to a second console by an image cable. In turn, the second console is connected to a screen device by a screen cable in order to display the captured image. The screen device may be arranged at the ceiling of the operating room or integrated in the surgical tower, e.g., next to the second console.

The optical device of the prior art includes a light-cable connector adapted for connecting a light cable. The light cable is a fiber optic cable that may include a plurality of optical fibers. The light cable is configured to transmit visible light from a first console to the optical device. The first console may be arranged in the surgical tower. In this way, visible light is provided to an interior portion of the patient, e.g., the human body. Therefore, the acquired captured image is illuminated so the surgeon may see as required the interior portion of the patient, e.g., the human body, at least when performing the surgery.

The image cable connecting the arthroscopy device to the user device (e.g., the second console of the surgical tower) may be uncomfortable by the surgeon when performing the surgery. In fact, cables or wires connecting the prior art arthroscopy device to the user device may be uncomfortable by the surgeon when performing the surgery. Said cables or wires may be the image cable, the light cable, and optionally a conduit or tube for providing a water solution to remove optical obstructions when acquiring the captured image.

Further, the arthroscopy devices are protected by a plastic cover or sheath. That is, prior art arthroscopy devices are enclosed by said plastic cover for sterility purposes, since certain surgical instruments, such as an arthroscope, cannot be sterilized directly (e.g., by submersion or autoclaving under high heat and pressure), so they need to be enclosed in a sterile plastic cover. This ensures sterility for devices that cannot be sterilized by conventional dry heat techniques.

The cables or wires, conduits or pipes, and the plastic cover together form a bulky system that becomes uncomfortable for the surgeon to manage. Once the surgery has started, the surgeon holds the prior art arthroscopy device, while the image cable, the light cable, and the conduit are continuously dragged over the surgical field throughout the surgery. In addition, since the surgical system is in constant use during the surgery, these components tend to become tangled and create a significant mess over time. Also, these cables, wires or conduits are not sterilizable, so they must also be wrapped in the plastic cover, which increases the preparation time of the prior art surgical device (e.g., the arthroscopy device).

Additionally, the surgeon during the surgery cannot touch the console, since it is not sterile, therefore, it forces him or her to ask a nurse to modify the different parameters on the console. These consoles are not easy to use if the nurse does not have technical knowledge. It is common that, in the event of any technical problem during surgery related to the arthroscopy device or the consoles connected thereto, it becomes necessary to notify personnel external to the operating room team, resulting in the need to stop surgery with all the inconveniences that entails. These technical problems (e.g., image adjustment, problems with image quality, screen connectivity, white contrast, poor connections between consoles, malfunctioning of a light cable, etc.) far from being exceptional, they could rather be considered usual. In use, since the surgery tower typically includes two or more consoles, all consoles must be compatible with each other. As a result, if one console is faulty and needs replacement, it may require replacing all the consoles of the surgery tower.

Prior art surgical systems may also be provided with an image recording device that is configured to record video/images, for example, on a USB stick. The image recording device is a console (i.e., third console) located in the surgery tower. Most image recording devices do not allow internal storage, so the surgeon who desires to record any images must insert a USB storage device (e.g., pen drive) before the start of the surgery. Compatibility problems between the image recording device and the USB storage device are also common. On the other hand, hospitals do not usually have enough image recording devices for all their arthroscopy devices so they have to be moved from one surgery room to another or simply the surgical systems do not have the possibility to record images.

Furthermore, the light cable is often subject to damage, resulting in a gradual loss of brightness. It is not uncommon for the light cable to require replacement one or more times during surgery.

In the known prior art, the optical device is only compatible with a specific image cable. Similarly, the image recording device is only compatible with a specific image recording cable. If a breakdown occurs, either in the optical device, light cable, image cable, or a console, then only a specific replacement may be compatible. That is, not any optical device, light cable, image cable, or console may replace the broken one. The lack of compatibility between of the different devices provided by different manufacturers represents an additional cost for the hospitals and the need to maintain specialized personnel for their repair.

It would be therefore desirable to provide an improved surgical device, system, and related method in order to obviate the above-mentioned drawbacks and to provide advantageous solutions to the shortcomings in the prior art.

### SUMMARY

It is an object of the present invention to provide a wireless surgical system. The wireless surgical system comprises a surgical device, and one or more user devices. The surgical device may be a wireless surgical device. That is, a cable-free surgical device (i.e., the surgical device does not comprise a cable or wire coming out therefrom to transmit at least image data to the one or more user devices). In fact, the one or more user devices and the surgical device are configured to wirelessly transmit data to each other, i.e., without a physical component such as a wire or cable. In other words, the surgical device and at least one user device are connected to each other in a wireless manner.

The surgical device comprises: an optical device, a light connector, and an image sensor.

The optical device is or comprises a lens carrier. The optical device includes or receives an optical body. In turn, the optical body may comprise one or more lens bodies.

The light connector is configured at least to receive visible light such that, in use, the surgical device illuminates an interior portion of an anatomic body. It is preferred that the surgical device further comprises a light-supply device in light connection to the light connector. The light-supply device includes a light source. In use, the light-supply device is intended to illuminate the interior portion of the anatomic body. For this, the light-source is configured to generate visible light (i.e., illumination light).

The image sensor is in optical communication with the optical body (e.g., the lens body). The image sensor is configured to acquire a captured image of the interior portion of the anatomic body illuminated by the visible light. Particularly, the image sensor generates raw image data (i.e., video data) that corresponds to the captured image.

The surgical device is used for minimal-invasive surgery. In particular, the surgical device may be a wireless arthroscopy device. Other types of surgical devices are of course possible such as a wireless arthroscopy, fluoroscopy, laparoscopy, stereoscopy, retinoscopy, gastroscopy, colonoscopy, lecanoscopy, and so on.

The anatomic body may be or comprise a piece or body at least to be explored therein for medical (e.g., surgical) and/or veterinary purposes. It may also serve educational purposes in the fields of medicine and/or veterinary studies. For example, the anatomic body may be a living being such as a person. A death person may also be understood as the anatomic body. The living being may be a human being such as a patient requiring surgery. The surgical device assists in diagnosing and treating conditions of the anatomic body, e.g., joint problems. It enables the surgeon to view the interior portion of the anatomic body without the need for a large incision.

The optical device may include an elongated portion or scope defining a longitudinal axis. The elongated portion is intended to be at least partially introduced into the anatomic body. The optical device may include one or more optical fibers. For example, the optical body is surrounded by a fiber-optic sleeve (i.e., a thin fiber-optic tube). Further, the fiber-optic sleeve may be surrounded by a protective outer-sleeve. The elongated portion may have an outer diameter of between 2 and 6 millimeters. At one end of the optical device, a video connection is arranged, wherein a lens body is arranged at the patient end. The lens body (e.g., an optical body) may be angled (e.g., between 30° and 70°) relative to the optical axis to allow a larger field of view when the scope is rotated. In use, the user may rotate the scope manually about the longitudinal axis. The lens body may be attached to or (form an integral) part of the optical body.

The optical device may be adapted for transmitting the visible light (i.e., illumination light), e.g., produced by the light-supply device, into the interior portion of the anatomic body through the one or more optical fibers. For this, the light connector may comprise a thread connection. The light-supply device may be attached to the optical device by use of the thread connection. It is preferred that the light-supply device may be arranged substantially perpendicularly to the optical axis. As explained, the optical device is also adapted for collecting the illuminated image inside the interior portion of the anatomic body and project it to the image sensor through the optical body (e.g., the lens body).

The image sensor may be part of a (miniaturized) camera device (for surgeries). In fact, the camera device comprises the image sensor being in optical communication with the optical body (e.g., the lens body). The camera device is thus connected to the optical device and the controller. The captured image acquired by the camera device is received by the controller. It is preferred that both the light-supply device and the camera device do not comprise connecting cables or wires coming out from the surgical device.

The surgical device further comprises the following: a circuit board, a controller, and a transceiver. These parts or components make it possible for the surgical device to operate wirelessly, i.e., to emit and/or receive at least image data derived, at least in part, from the raw image data without any cable, or wire, or physical component extending from the surgical device.

The circuit board may be a printed circuit board or PCB. It is possible that the circuit board comprises two or more printed circuit boards, for example, connected to each other. Alternatively, the circuit board may be a single printed circuit board. In any case, the circuit board may be adapted for receiving electronic components as required.

The controller may be understood as a single (board) computer. The controller may comprise a single electronic control unit or, alternatively, two or more control units that may be integrated together or not, or, in the same location or not. The controller may include an encoder. The encoder, as explained further below, may be a software encoder or preferably a hardware encoder. In any case, the controller is attached to the circuit board. This is, the circuit board comprises the controller. Preferably, the controller includes one or more of a CPU, RAM memory, ROM memory, and a network interface circuit.

The light-supply device may optionally be electrically connected to the circuit board, particularly, to the controller. In operation, the controller may optionally be configured at least to generate an illumination light signal to the light-supply device for the light source to generate visible light so as to illuminate an interior portion of the anatomic body. It is absolutely possible that the light-supply is not electrically connected to the circuit board. For example, the light-supply device may incorporate a battery to provide electric power to the light-supply device. Further, the light-supply device may include a switch device to turn on and off the supply of light, i.e., illumination light to the interior portion of the anatomic body.

It is preferred that the surgical device further comprises a power source configured to supply electric power to the controller, the image sensor, and optionally to the light-supply device. More preferably, the power source is a single battery unit, for example, having a voltage higher than 1.5 volts, particularly, of between 1.5 volts and 20 volts.

Preferably, the surgical device further comprises a housing assembly that is adapted for enclosing the circuit board, the controller, the transceiver, and the power source. The housing assembly protects said components from external agents or impacts. The housing assembly may be made of polymeric material, for example, polyamide material such as Nylon as it has good biocompatible qualities. The housing assembly comprises a first housing part and a second housing part that fit together defining an inner portion therein to receive the circuit board, the controller, the transceiver, and the power source.

It is preferred that the surgical device further comprises an electronics support. In fact, the camera device includes said electronics support. In any case, the electronics support is arranged substantially perpendicular to the optical axis. The image sensor is attached to the electronics support. Furthermore, the circuit board may be arranged substantially perpendicular to the electronics support, thereby rendering the surgical device more compact and substantially improving ergonomics.

An image data transmission medium such as a high-bandwidth image data transmission medium may be further provided. The (high-bandwidth) image data transmission medium may be configured to electrically connect the image sensor and the controller to each other for transmitting the raw image data, or image data derived therefrom, to the controller (in a highspeed manner). In this way, the controller may be received with the raw image data or any image data at least partially derived therefrom. More optionally, the high-bandwidth image data transmission medium is a flat flexible cable, or a flexible printed circuit board, or a CSI cable (Camera Serial Interface cable).

The transceiver may be attached to or be (an integral) part of the circuit board. In use, the transceiver is in electrical connection to the controller. The transceiver is configured to emit and/or receive data out of the surgical device, e.g., to one or more user devices.

The transceiver may comprise a transmitter and a receiver. Therefore, it is absolutely possible that the transmitter and the receiver are integrated into a one single-device such as the transceiver.

In operation, the controller may be configured at least to convert (e.g., encode) the raw image data (generated by the image sensor) (or any image data at least partially derived from the raw image data) into a video encoding format data. The video encoding format data may be or comprise compressed video data. It is preferred that the video encoding format is protocol H264. Other protocols may be used such as H265, H266. The size of the video encoding format is reduced compared to the raw image data. Protocol H264 has been found to be preferred for the present invention, as the issue of low latency in the surgery device does not allow time for complex encodings.

In order to be able to work with the raw image data in image format, an image encoding is performed. Image quality may be sacrificed to reduce the dimensions of the raw image data. For example, in H264, there is a reduction of the size of the raw image data by 90%. The main reasons for using a video encoder to convert the raw image data into the video encoding format date are the following:
- reduce the size of the (image) data so that they can be managed by the controller;
- reduce processing and transmission time since size of the (image) data is reduced, for example, by 90% with H264; and
- increase compatibility with other devices such as the one or more user devices. Most electronic devices that are operated with images do not have native support with raw image data. The use of the encoder for the raw image data allows compatibility with a wide variety of the one or more user devices.

Further, in operation, the controller is configured at least to convert (e.g., packetize or transcode) the video encoding format data (e.g., H264 format) into a Real-Time Protocol (RTP) such as WebRTC. In use, it enables real-time communication between the controller and one or more user devices, as well as multimedia data exchange, as explained further below.

Simply coded videos (e.g., in protocol H264 format) are not structured in a way that are able to self-manage the transport of packets, error checks, and synchronization among others. Also, for security reasons, sending data in a video encoding format is not acceptable either. As the present invention requires real-time operation, the controller encodes the video encoding format data (in H264) in a specialized protocol for real-time transport over wireless networks.

Wires or cables are thus advantageously avoided between the surgical device and the one or more user devices. The use of WebRTC is been found advantageous mainly since apart from giving low latencies, it is encrypted to guarantee secure communication, necessary in this communication. It may also be played by web browsers, as well as by most electronic devices and media players. As explained, it is an aim of the present invention that the surgery device remains compatible with as many user devices as possible.

The surgical device may be configured to create a local network. The local network is a wireless local-area network (WLAN). In use, the local network may be a group of at least the surgical device and the one or more user devices that form a network based on radio transmissions rather than wired connections. Preferably, the local network is a Wi-Fi network.

Preferably, the controller is configured to create the local network. More preferably, the controller may be configured to operate the transceiver as an access point (AP) to create the local network. Therefore, the controller may be configured to create a local network by acting as a wireless access point (AP), allowing the one or more user devices to communicate with the controller one another and vice-versa without needing external internet access. This is achieved by configuring the controller to operate as a DHCP server (explained below), assigning IP addresses to connected devices, and managing network traffic. The local network is thus defined as a closed system of interconnected devices within a limited area, where data can be exchanged between devices without reliance on external networks, such as the Internet.

The controller may comprise an identity server and a multimedia server. Both the identity service and the multimedia server are arranged within the surgical device, the surgical device being a one-single device. The controller (e.g., the identity service and the multimedia server) is arranged within the housing assembly.

The identity server or authentication server may be configured to assign dynamic identities to the one or more devices connected to the local network. Preferably, the controller (e.g., identity server) is configured at least to automatically assign Internet Protocol (IP) addresses to the one or more user devices to respond at least to a broadcast query by one or more user devices. The controller (e.g., identity server) may optionally provide at least one default gateway. The default gateway (address) is the point through which all communications of the local network may pass.

It is preferred that the identity server is configured to verify a data request to give access to the one or more user devices. In this way, the one or more user devices may have access to the surgical device, e.g., the controller, when approved. More preferably, the identity server is the DHCP server.

Preferably, the local network operates approximately at 2.4 GHz. It is preferred that the dynamic identity assignment performed by the identity server is facilitated through a local network connection established via Wi-Fi. However, it is possible to use alternative technologies such as Bluetooth protocol (e.g., Bluetooth 5.0).

It is not necessary that the controller comprises the gateway. As at least some communications of the surgical device are assigned within the same network a gateway is not strictly necessary for data transmission between the surgical device and one or more user devices. However, it is preferred that the controller comprises the gateway even if it does not need to communicate outside the local network. A reason is that the one or more user devices may expect a default gateway to be assigned via DHCP, otherwise may lead to connectivity issues or warnings. Further, some network services and protocols may rely on the presence of a gateway for proper operation, even within a local network. The controller of the surgical device runs the DHCP server to manage connected devices efficiently. Assigning it as the gateway IP simplifies network configuration and ensures consistent communication paths. As the Wi-Fi hotspot creator, the controller effectively acts as the network's central hub. Assigning it the gateway IP reinforces its role in managing network traffic.

The controller (e.g., the multimedia server) may be configured at least to transmit the image data derived at least partially from the raw image data simultaneously and synchronously to the one or more user devices in a wireless manner through the transceiver once the one or more user devices is/are connected to the local network. In this context, simultaneous and synchronization is referred to at the level of the surgical device. However, if it is at the level of the wireless surgical system (i.e., including the one or more user devices), then it may not be necessarily simultaneous and synchronization, since the present invention does not control the synchronization of both the one or more user devices and the surgical device. In fact, (image) data on the one or more user devices may not be synchronized.

It is preferred that the multimedia server is configured to receive video encoding format data (based on the raw image data) and convert (e.g., packetize) it into a Real-Time protocol to be transmitted simultaneously and synchronously to the one or more user devices in a wireless manner through the transceiver once the one or more user devices is/are connected to the local network, i.e., the one or more user devices are within the network. That is, the multimedia server may receive data in H264 format. For this, the raw image is transformed into H264 preferably with hardware encoding and sent to the multimedia server for packetizing (transcoding) from H264 to RTC.

The multimedia server is a system that may comprise hardware, software, or a combination of both. It is configured to store, process, and deliver multimedia content, such as video, audio, and images, to the one or more user devices over the local network. As explained further below, the multimedia server may be configured to handle requests from the one or more user devices, manages access to media files, and streams content using various protocols and formats to ensure efficient and reliable delivery.

Particularly, the controller configured as a multimedia server may perform the following functions:
- multimedia data handling: the controller captures, receives, and processes multimedia data from one or more user devices. It converts, packetizes, encodes or transcodes the multimedia data into appropriate formats suitable for transmission to the one or more user devices (e.g., RTC);
- client communication interface: the controller provides an interface for the one or more user devices to send data requests and receive multimedia data. This interface supports multiple communication protocols and streaming technologies to facilitate compatibility with various user devices;
- streaming and delivery: the controller transmits multimedia data to one or more user devices using streaming methods. This includes support for real-time communication protocols and adaptive streaming technologies;
- user verification and security: the controller implements user authentication mechanisms to verify and authorize access from the one or more user devices. It manages user credentials and enforces access control policies to secure the multimedia data;
- simultaneous transmission: the controller is capable of transmitting multimedia data simultaneously to multiple user devices (i.e., two or more user devices); and
- dynamic client management: the controller supports dynamic assignment of identities and allows the one or more user devices to connect and disconnect seamlessly. It manages network resources to accommodate changing sets of connected devices without interruption to the service.

In short, the video encoding format data is in H264 format, and the Real-Time Protocol is WebRTC.

The present invention may provide at least the following advantages:
- simplified network and configuration, resulting in a more robust surgical system due to fewer components in the device's chain of operation, leading to fewer potential issues;
- increased robustness, as fewer components in the device's chain of operation reduce the likelihood of malfunctions;
- reduced cost due to reduced infrastructure requirements.
- faster performance with reduced latency;
- direct access to data transmission, creating direct access, very optimal for streaming; and
- enhanced security against computer attacks, as the data are not exposed to the internet, resulting in a more secure wireless surgical system.

In other words, the controller (e.g., the multimedia server) is further configured to send at least image data packetized with the Real-Time protocol to the one or more user devices in a wireless manner by the transceiver. For example, the controller has the video in H264, the multimedia server takes it, splits it into packets organized using the Real Time Protocol (RTP), for example, WebRTC. WebRTC establishes a direct connection with the one or more devices that make the request for it. Once verified, a "peer-to-peer" connection is created where latency is reduced. In operation, the one or more user devices receive the packets, reconstruct the media file (i.e., media data or content), and decode the H264 data that was inside the packet. This decoding may also be accelerated by hardware encoder employed by the one or more user devices.

An advantage is that the wireless connection between the surgical device and the one or more user devices do not depend on hospital Wi-Fi or any other external infrastructure (Internet). An aim of the present invention is to provide the user with everything necessary to use the one or more user devices as required. It may be used therefore in humanitarian missions, armies or in remote places. The local network allows not only be 100% independent of any pre-existing infrastructure, but also creates a more controlled environment where the security of the data passing through it is guaranteed.

Since the surgical device and the one or more user device are on the same local network, it is possible to minimize the number of "hops" packets have to make from the surgical device to the one or more user device. Thus, the total delay of the system can be better controlled.

The fact of integrating all the tasks, functions or operation of the controller (on a single board) allows to minimize the total latency of the wireless surgical system (from the time light enters through the lens body until the user sees it in the form of an image on the one or more user devices). In fact, the latency of the wireless surgical system is equal to or lower than 250 milliseconds which is the human reaction time (i.e., surgeon reaction time).

Further, it allows a variety of user devices to be wirelessly connected to the surgical device. By making use of the transmitter (e.g., antenna) and creating the local server (e.g., DHCP server) on the controller (this is a reason why the controller includes a CPU, RAM memory, ROM memory, and a network interface circuit), a creation of the local network is completed. As explained, the identity server is in charge of assigning IP addresses to the two or more user devices that connect to it (i.e., the local server allows the possibility of having as many devices on the local network as the IP range of the local network allows). This is, the surgical device may be connected in a wireless manner with two or more user devices. The identity network allows all nearby devices (i.e., two or more user devices) to be able to connect to the multimedia server to have access to a broadcast video. This is particularly useful in scenarios involving a first users (e.g., a surgeon using the first user device) and a second user (e.g., students in a classroom viewing for academic purposes using a second user device) who are observing the interior portion of the anatomic body.

The above-mentioned power source is adapted for supplying electric power to the controller to operate the local network, the identity server, and the multimedia server.

As explained above, the one or more user devices are configured to access the controller of the surgical device, e.g., to connect to the controller. In fact, the one or more user devices receive the image data packetized with the Real-Time protocol (e.g., WebRTC). Further, the one or more user devices may be configured to decode the image data encoded with the Real-Time protocol (e.g., WebRTC) into the video encoding format (e.g., protocol H264). In this way, the one or more user devices may be capable of displaying the video encoding format.

It is preferred that the one or more user devices is configured to send a configuration request such as a HTTP request to the surgical device. In operation, the controller may be capable of changing at least one configuration value in real time and on-the-fly such as image contrast, brightness, resolution, and/or frame rate.

It is preferred that the one or more user devices may be configured to send a recording request such as a HTTP request to the surgical device. In operation, the controller of the surgical device is capable of recording image data. It includes saving the image data on a pen drive, for example.

The one or more user devices may be a head-mounted device such as augmented reality (AR) glasses. Also, the one or more user devices may include tablets, smart phones, or computers (using a GPU or Graphic Processing Unit, a graphics card). The user may be a doctor, physician or surgeon.

### BRIEF DESCRIPTION OF THE DRAWINGS

Non-limiting examples of the present wireless surgical device, system, and method will be described in the following, with reference to the appended drawings.

In the drawings:
Figure 1 is an illustration of a prior art surgical device that illustrates a bundle of tubes, cables, or wires coming out from the prior art surgical device;
Figure 2 is an illustration of a wireless surgical system according to an example of the present invention that illustrates a wireless surgical device, a surgery tower that includes a screen device, and surgeons looking at the screen device, wherein the wireless surgical device and the surgery tower are wirelessly connected to each other;
Figure 3 is a perspective view of the wireless surgical device according to an example of the present invention;
Figure 4 is an exploded view of the wireless surgical device;
Figure 5 is a perspective view of the wireless surgical device without a cover housing allowing the power source, and the circuit board to be shown;
Figures 6 is a top view of the wireless surgical device without a cover housing allowing the components arranged inside the housing assembly to be shown;
Figure 7 is a top view of the circuit board of the wireless surgical device connected to the camera device;
Figure 8 is a schematic illustration of different transformations and transmissions of image data;
Figure 9 is a schematic illustration conceptually similar to figure 8 but with greater level of detail; and
Figure 10 is a schematic illustration that illustrates the wireless surgical system and a related method.

### DETAILED DESCRIPTION OF THE EXAMPLES

With reference to the non-limiting examples shown in the figures of the drawings, a wireless surgical device, and system that includes said a wireless surgical device is described herein as well as a related method.

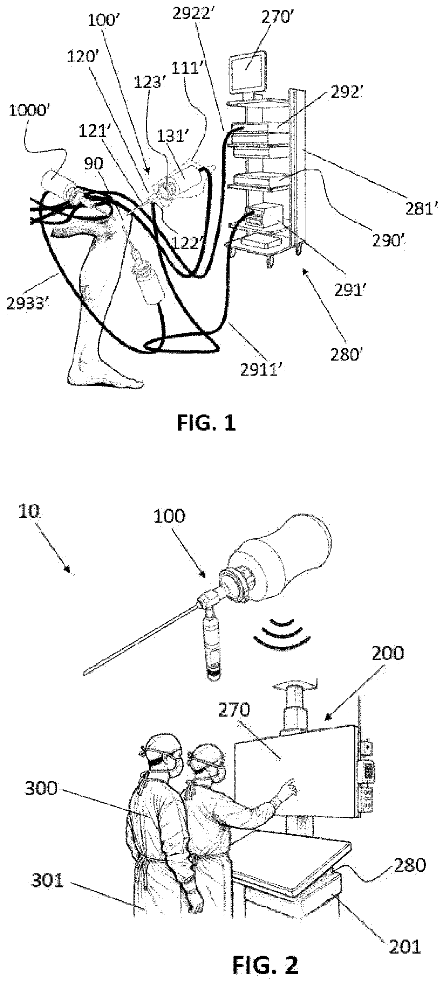

Figure 1 shows a prior art surgical device 100' being particularly an arthroscopy device.

The prior art surgical device 100' comprises an optical device 120', and a video camera 131'. In turn, the optical device 120' includes an elongated portion 121' or scope, a light-cable connector 122', and a video connection 123'. The light-cable connector 122' is adapted for receiving a light cable 2911'. The video connection 123' is adapted for connecting to the video camera 131'. The video camera 131' includes an image sensor in optical communication with an optical lens of the elongated portion 121'.

Figure 1 also shows an arthroscopy tower 280' comprising a support frame 281' adapted for receiving a plurality of consoles 290'. A first console 291' is used for providing visible light to the prior art arthroscopic device 100' through a light cable 2911'. That is, said first console 291' is particularly configured to generate visible light (i.e., illumination light) to be transmitted through the light cable 2911' to the optical device 120'. In this way, an interior portion of the anatomic body 90 is illuminated as required. A second console 292' of the plurality of consoles 290' is used for receiving image data. That is, said second console 292' is particularly configured at least to receive image data from the prior art arthroscopic device 100' (e.g., from the video camera 131'). Further, the arthroscopy tower 280' comprises or is connected to a screen device 270' to provide the surgeon with a video captured by the prior art arthroscopic device 100'. Said captured video corresponds to a video of the interior portion of the anatomic body 90.

As shown in figure 1, cables or wires 2911', 2922', are coming out from the prior art surgical device 100'. In particular, figure 1 shows that the light cable 2911', and the image cable 2922' are coming out from the prior art surgical device 100', wherein a tube 2933' is coming out from a fluid discharger 1000' for surgeries. It is possible that the prior art surgical device 100' and the fluid discharger 1000' for surgeries are integrated into a single device. The light cable 2911' is configured to transmit light generated by the first console 291' to the prior art surgical device 100'. The image cable 2922' is configured to transmit image data to the second console 292'. The tube 2933' allows liquid containing water to flow through and be ejected into the interior portion of the anatomic body 90 as required. During surgery, the surgeon holds the prior art arthroscopic device 120' that is protected by a sheath or plastic cover 111' (depicted by a dashed line), which is usually bulky. Further, tubes, cables or wires 2933', 2911', 2922' are continuously dragged over the surgical field. As surgical portals are constantly manipulated during the surgery, a significant mess of these tubes, cables, or wires 2933', 2911', 2922' often occurs in a short period of time.

The following explanation of figures 2 to 10 shows non-limiting examples of the present invention.

Figure 2 illustrates a wireless surgical system 10 comprising a wireless surgical device 100 that will be explained below, and one or more user devices 200. In particular, the one or more user devices 200 (e.g., a first user device 201) is or comprises a surgical tower 280. As shown, the first user device 201 includes a screen device 270. The first user device 201 (e.g., the surgical tower) may comprise one or more consoles as explained above for the prior art. In any case, the wireless surgical device 100 and the first user device 201 is wirelessly connected to each other. Figure 2 also illustrates a user 300 who is a surgeon. The user 300 interacts with the first user device 201 (e.g., watches the screen device 270, and manipulates the first user device 201 as required).

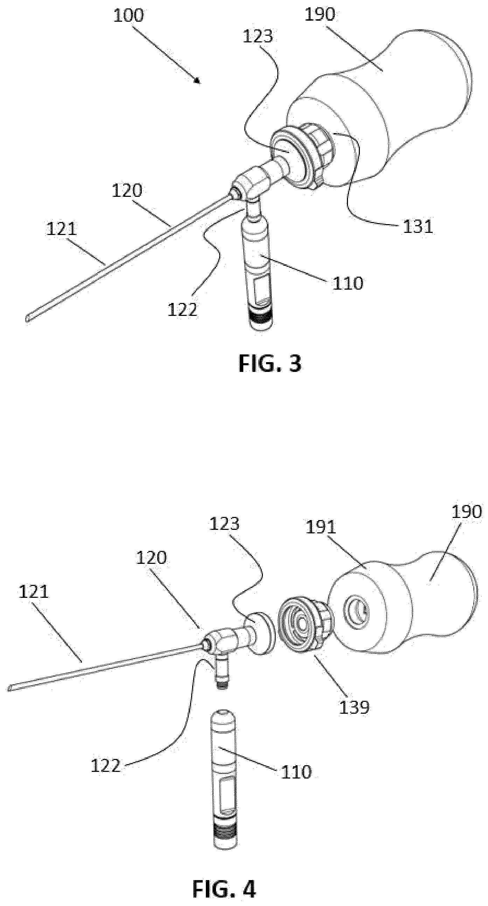

Figures 3-6 show the wireless surgical device 100 of an example of the present invention. In particular, the wireless surgical device 100 is a wireless arthroscopic device.

The wireless surgical device 100 comprises an optical device 120, and a video camera 131.

In turn, the optical device 120 (or lens carrier) includes an elongated portion 121 or scope, a light connector 122, and a video connection 123.

In examples, the light connector 122 is adapted for receiving the light-supply device 110 (shown in figures 2-6), or a light cable 2911' such as the one explained in the prior art (see figure 1). In use, visible light (i.e., illumination light) is provided to the optical device 120. The light-supply device 110 is in light connection to the optical device 120 via the light connector 122. The light connector 122 includes a thread. The light-supply device 110 comprises a light source to generate visible light so as to illuminate the interior portion of the anatomic body 90. As shown in figures 2-6, the light-supply device 110 is arranged substantially perpendicular to the elongated portion 121. In the shown examples, the light-supply device 110 is a light torch.

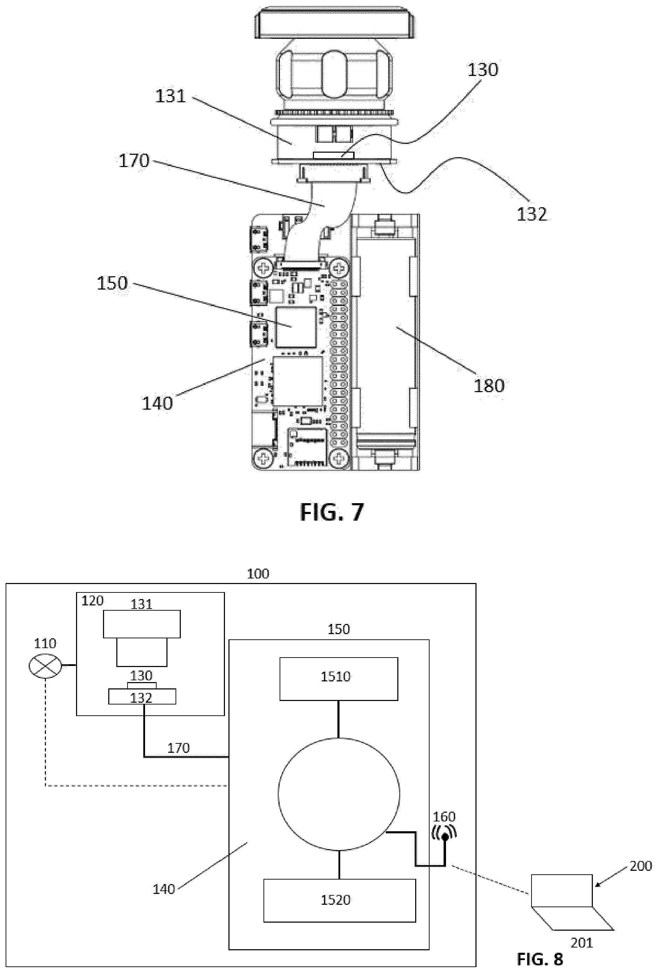

The video connection 123 is adapted for connecting to the video camera 131. The video camera 131 includes an electronics support 132, and an image sensor 130 (see figure 7). The image sensor 130 is attached to the electronics support 132 in optical communication with the optical device 120. In use, the image sensor 130 is configured to acquire a captured image of the interior portion of the anatomic body 90 illuminated by the visible light (i.e., illumination light). The image sensor 130 is configured to generate raw image data 1310 of the captured image.

The wireless surgical device 100 is used for a surgical procedure that uses a video camera 131 (e.g., a fiber-optic camera) to diagnose and treat problems of the anatomic body 90, e.g., joint problems.

During surgery, the surgeon inserts the optical device 120 through a small incision. In particular, the elongated portion 121 is inserted to the anatomic body 90. In the shown example, the anatomic body 90 is a knee of a human being. The elongated portion 121 is a narrow tube defining a longitudinal axis. The elongated portion comprises an optical body (e.g., a slim telescope that, for example, may be about 25cm in length, arranged inside the narrow tube). The optical body comprises a plurality of lens bodies arranged one after the other in a direction of the longitudinal axis. The optical body (e.g., an optical lens) is optically connected to the video camera 131 (e.g., in optical communication with the image sensor 130). The optical body is surrounded by a thin fibre-optic tube.

The video connection 123 is arranged at or proximate to a first end 120A of the optical device 120, wherein at least a lens body is arranged at or proximate to a second end 120B (i.e., "patient" end). Said lens body may be angled between of 30° and 70° relative to the longitudinal axis O to allow a larger field of view as the elongated portion 121 is rotated about the longitudinal axis O.

During surgery, the view of the interior portion of the anatomic body 90 is transmitted wireless to the first user device 201, e.g., the screen device 270. It allows the surgeon 301 to see the interior portion of the anatomic body 90.

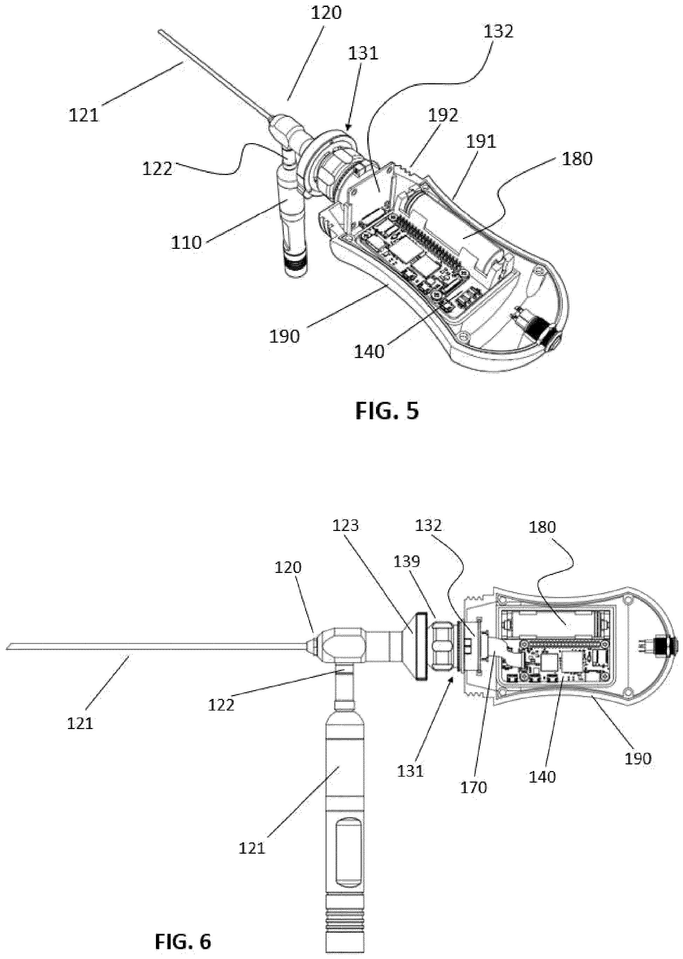

Figures 3-6 show that the wireless surgical device 100 further comprises a housing assembly 190 that is adapted for enclosing a circuit board 140. The electronics support 132 is arranged substantially perpendicular to the longitudinal axis O. The circuit board 140 is arranged substantially perpendicular to the electronics support 132 (see figures 5 and 6). In examples not shown, the circuit board 140 and the electronics support 132 are integrated into a single (one-unit) component (e.g., in the same printed circuit board). The circuit board 140 has a larger surface area than the electronics support 132, for example, at least twice its size.

Figures 7-10 illustrate that the wireless surgical device 100 further comprises a controller 150 and a transceiver 160 both directly attached to or being an integral part of the circuit board 140.

The transceiver 160 comprises a transmitter 161 and a receiver 162 being in electrical connection to the controller 150. In examples, the transmitter 161 and the receiver 162 are integrated into a one-single device such as the transceiver 160.

As illustrated in figures 6-10, the controller 150 is directly attached to the circuit board 140. This is, the circuit board 140 includes the controller 150. Further, the controller 150 is electrically connected to the image sensor 130 (see figure 7).

In particular, figure 6 shows that the optical device 120 (e.g., the lens body) and the image sensor 130 (see figure 7) forms a camera device 131. The camera device 131 is particularly for surgeries. In fact, the camera device 131 is a miniaturized camera device, for example, for arthroscopies.

As shown in figures 6-7, the wireless surgery device 100 further comprises a high-bandwidth image data transmission medium 170. Particularly, the high-bandwidth image data transmission medium 170 is CSI cable. It electrically connects the controller 150 and the camera device 131, e.g., the image sensor 130, to each other for transmitting the raw image data 1310.

Figures 5-7 show that the wireless surgery device 100 comprises a power source 180 configured to supply electric power to the controller 150, the image sensor 130, and optionally to the light-supply device 110. In particular, the power source 180 is a single battery unit, for example, having a voltage of between 1.5 volts and 20 volts such as 5 volts. In use, the power source 180 provides sufficient electrical power to operate the controller 150 and/or the light-supply device 110 for more than two hours, for example, more than four hours.

In examples, the controller 150 is further configured to monitor and control a voltage, current, and/or temperature of the power source 180 to ensure consistent and safe operation during surgical procedures. Optionally, the wireless surgery device 100 includes a protection module adapted to prevent overcharging, overdischarging, and overheating, thereby enhancing reliability and lifespan of the power source 180. Additionally, the wireless surgery device 100 may comprise a communication interface arranged to transmit real-time power status to the controller 150, enabling precise energy management and uninterrupted operation. It is designed to ensure optimal performance and reduce the risk of power loss during surgical procedures.

Figures 5 and 6 show that the housing assembly 190 is adapted for enclosing the circuit board 140, the controller 150, the transceiver 160, and the power source 180. The housing assembly 190 comprises a first housing part 191 (see figure 5) and a second housing part adapted for being attached to each other to define an interior space therein. The second housing part is particularly a cover part. The housing assembly 190 (e.g., the first housing part 191 and/or the second housing part) comprises a thread connecting portion 192, wherein a housing connecting part 191 (see figure 4) has a thread structure adapted to engage the thread connecting portion 192. As shown, the housing connecting part 191 is part of the housing assembly 190. In this way, the housing assembly 190 is mounted without the need for fasteners, welding, or other types of attaching means.

Figure 4 shows that the wireless surgery device 100 further comprises a C-mount endescope adaptor 139. The C-mount endescope adaptor 139 is adapted for connecting the optical device 120 and the camera device 131. Particularly, the C-mount endescope adaptor 139 has a front portion that includes an aperture adapted for receiving the video connection 123, wherein a back portion includes a connecting portion enabling optical communication with the image sensor 131. The C-mount endescope adaptor 139 includes a diafragma-like system that opens in order to slide in the endoscopic lens.

Furthermore, the wireless surgical device 100 is also provided with at least an ON/OFF button connected to the controller 150 via a GPIO (not shown). In particular, the button includes a latching LED illuminated waterproof button that will be illuminated while the surgical device is ON, acknowledging its state to the user 300.

In figures 8-10, the controller 150 comprises an identity server 1510 and a multimedia server 1520. Further, the controller 150 is configured at least to transmit image data derived at least partially from the raw image data 1310 to the first user devices 201 in a wireless manner through the transceiver 160.

The controller 150 is configured to create a local network. In particular, the local network operates approximately at 2.4 GHz. This transmitter 160 is used to create a signal range.

The identity server 1510 is an authentication server configured to assign dynamic identities at least to the first user device 201 connected to the local network, and provide a default gateway.

The multimedia server 1520 is configured at least to transmit the image data derived at least partially from the raw image data 1310 simultaneously and synchronously to the first user device 201. It only occurs when the first user device 201 is connected to the local network. The identity service 1510 and the multimedia server 1520 are both arranged within the housing assembly 190, the wireless surgical device 100 being a one-single device.

Figures 8-10 illustrate the wireless surgery device 100 and one user device 201. The controller 150 of the wireless surgery device 100 receives the raw image data 1310 generated by the image sensor 130. The controller 150 includes an encoder (either software-based or hardware-based) to encode the raw image data 1310 so as to convert it into a video encoding format data 1320. The video encoding format data 1320 uses protocol H264. The multimedia server 1520 is configured to receive the video encoding format data 1320 and packetize (transcode) it into a Real-Time protocol 1330 to be transmitted (simultaneously and synchronously) at least to the first user device 201 by the transceiver 160 after the first user device 201 is connected to the local network. In particular, the Real-Time protocol 1330 is WebRTC.

The receiver 261 of the first user device 201 is configured to receive the video encoding format data 1320, which is packetized using the Real-Time protocol 1330 and transmitted by the transmitter 161. It only occurs when the first user devices 201 is connected to the local network. The first user device 201 is configured to convert the received video encoding format data 2610, which is packetized using the Real-Time protocol, into a video encoding format data 2610' that is not packetized. In this way, the video encoding format data 2610' of the first user device 201 is displayed through a screen device 270. In examples not shown, the first user device 201 may be a wearable such as augmented reality glasses. If so, the video encoding format data 2610 is viewable by the first user 301. As explained, the first user 301 is a surgeon for example.

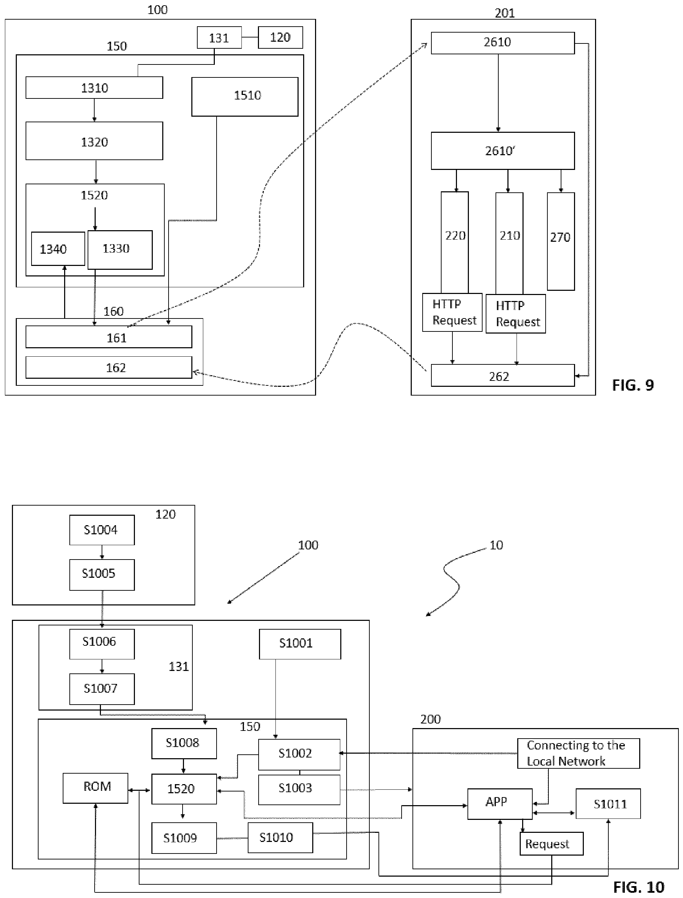

The first user device 201 includes a software application, i.e., app. The first user 301 interacts with the software application to generate a recording request 210 and/or a configuration request 220 (see figure 9).

The recording request 210 is a Hypertext Transfer Protocol (HTTP) request. The recording request 210 is sent by the transmitter 262 of the first user device 201 to the transceiver 160, e.g., receiver 162, of the wireless surgical device 100. It only occurs when the user device 201 is connected to the local network. The transceiver 160, e.g., the receiver 162, is electrically connected to the controller 150, particularly, to the multimedia server 1520. In particular, the multimedia server 1520 comprises an application programming interface (API) 1340. The multimedia server 1520, e.g., the API 1340, is configured to receive the recording request 210. Then, the multimedia server 1520 is configured to store the video encoding format data. The video encoding format data may be stored internally within the multimedia server 1520 or externally, e.g., on a USB stick.

The recording-fetch request 210 is a fetch request. For this, an action is performed (e.g., by the API 1340) for the multimedia server 1520 to serve (e.g., provide) the video encoding format data to the first user device 201 via HTTP. Specifically, when the controller 150 needs to search for an archive generated by the multimedia server 1520 (e.g., the recording file), the process is carried out with an archive fetch, via HTTP, but in this case, the archive is returned. The controller 150 comprises a read-only memory (ROM) or non-volatile memory in order to store the recorded image data derived at least in part from the raw image data 1310. In particular, the stored image data is video encoding format data 1320, for example, in MP4 format. In non-limiting examples, the first user device 201 has access to the stored image data. It only occurs when the first user device 201 is connected to the local network. In this way, the first user 201 is able to watch again the recorded video with a simple click or interaction to the first user device 201 in real time and on-the-fly, and wherein the first user device 201 does not necessarily have to store the recorded video.

The configuration request 220 is a Hypertext Transfer Protocol (HHTP) request. The configuration request 220 is sent by the transmitter 262 of the first user device 201 to the receiver 162 of the wireless surgical device 100. It only occurs when the first user devices 201 is connected to the local network. The receiver 162 is electrically connected to the controller 150, particularly, to the multimedia server 1520. In particular, the multimedia server 1520 comprises the API 1340 that is configured to receive the configuration request 220. In use, the controller 150 is configured to generate an instruction for changing at least one configuration value in real time and on-the-fly of the image contrast, brightness, resolution, and frame rate. In this way, the first user 301 may change the image contrast, brightness, resolution, and/or frame rate with a simple click or interaction to the first user devices 201 in real time and on-the-fly.

With the same API 1340, parameters of the multimedia server 1520 can be changed. However, these parameters can either have only internal effects on the multimedia server (e.g., sending a video to be downloaded) or interact with external modules of the multimedia server 1520, such as a camera controller. The camera controller is configured to change at least one configuration value in real time and on-the-fly of the image contrast, brightness, resolution, and frame rate. That is, in the case of changing a parameter of the camera device, the HTTP request modifies it on the multimedia server, and then the multimedia server is configured to request a module responsible for communicating with the camera device 131 to apply those adjustments. The camera controller may be arranged at the electronics support 132.

The controller acts as a video-transmitting platform. It includes H.264 hardware encoding with up to 1080p resolution and 30 frames per second, which help to do fast video encoding without the need for a powerful CPU.

H.264 enables to efficiently compress high-quality video data without sacrificing much quality. H.264 does this by using several methods, including entropy coding, intra-frame prediction, and inter-frame prediction. While intra-frame prediction reduces spatial redundancy inside individual frames, inter-frame prediction takes advantage of temporal redundancy by forecasting object motion between frames. By giving shorter codes to symbols that appear often, entropy coding further compresses data.

When compared to older standards like MPEG-2, H.264 can achieve significant bandwidth savings because of these combined strategies. Also, it has extensive compatibility and adaptability.

The levels define restrictions on bitrate, frame size, and decoding power to guarantee interoperability among devices and networks. Every level is distinguished by a number that indicates its highest bitrate and frame size. For example, Level 4.1 specifies a maximum frame size of 1920x1080 pixels and a maximum bitrate of 62.5 Mbps. These limitations ensure that videos can be encoded and decoded efficiently and on devices with different processing power.

Profiles in H.264 define features and capability sets customized for certain devices or applications. These profiles provide a range of encoding tools and parameter combinations, including deblocking filters, entropy coding, and motion compensation among others. In particular, the Baseline profile offers assistance for aspects like interlaced video coding, motion compensation with variable block sizes, and context-adaptive variable-length coding. This profile is the most suitable profile for the present invention since it does not rely on other frames to start decoding, and it is the less complex of the three. To prove this, we have conducted different tests that will be shown in the following sections.

The controller includes a CPU, RAM memory, ROM memory, and a network interface circuit. In examples, the CPU is 1GHz, the RAM 512MB, and the wireless LAN 2.4GHz. It further includes a storage medium being particularly a microSD card. The high-bandwidth image data transmission medium 170 is a CSI-2. For this, a 2-lane MIPI CSI connector is used. The video encoding is a H.264, MPEG-4 encode (1080p30). Similarly, the video decoding is a H.264 video decoding (1080p30). The resolution of the image sensor 130 is 12.3 megapixels (4056 x 3040 pixels). The frame rate is at least 30 frames per second (fps). The lens mount is a C-mount. The camera device 131 is configured for manual focus adjustment.

With regards to the DHCP server, NetworkManager is a software utility designed to simplify the management of network connections on Linux-based systems. With a simple command, a local network on boot is created, where all the user devices can be connected in order to receive the live-feed from the camera device 131.

There are two selected DHCP server protocols for the two different use cases.

For the first use-case we have chosen WebRTC, a peer-to-peer protocol that offers ultralow latency. This protocol will be used to display the video-feed to the user doing the surgery or exploration since latency is a crucial factor for the usability of the project.

The second use-case is for external people (i.e., the second user 202) that want to display the video on their devices but do not interact directly with the device. For this, we have chosen HLS due to its high compatibility with internet browsers and, since it's not a peer-to-peer connection, it is more scalable and less resource-intensive for a bigger number of users. The first user device 201 is particularly a mobile application. React-native works with native components, meaning that the elements rendered in the interface will be rendered as native widgets, creating a smoother and more optimized application, closing on the performance of native apps, which require a great amount of time to develop and maintain.

Some other advantages of this framework are that it enables to do hot reloading, meaning that we can see the changes instantly on our application without the need of recompiling the app which might be time consuming.

The app allows splitting the view of the screen device 270 into three main section views. A first main section view, on which the user can readily navigate to the two primary functionalities provided by the application, namely initiating an exploration or accessing local multimedia files from prior surgeries.

Figure 10 illustrates an aspect of the present invention related to a method for operating a wireless surgical system comprising:
S1001: Pushing, by the user 300 (e.g., surgeon), the ON button. It is the start of the method. It may include start providing the illumination light.
S1002: Creating the local network by the controller 150.
S1003: Assigning, by the identity server 1510, at least one Internet Protocol (IP) address to the one or more user devices 200 (e.g., the first user device 201) connected to the local network to respond at least to a broadcast query by one or more user devices 200.
S1004: Inserting at least a portion of the surgical device 100, for example, the optical device 120 (e.g., the elongated portion 121), into the anatomic body 90.
S1005: Illuminating the interior portion of the anatomic body 90. For example, the light-supply device 110 produces visible light (i.e., illumination light). The visible light is provided to the optical device 120. The optical device 120 transmits the visible light through via the one or more optical fibers to the interior portion of the anatomic body 90.
S1006: Acquiring, by the camera device 131 (e.g., the sensor image 130), the captured image of the interior of the anatomic body 90 where the visible light is provided. The camera device 131 (e.g., the sensor image 130) generates raw image data 1310 or an image date derived therefrom.
S1007: Transmitting (e.g., by a high-bandwidth image data transmission medium 170) the captured image into the controller 150. The controller 150 is an integral part of the wireless surgical device 100.
S1008: Converting (e.g., encoding) the raw image data 1310 into a video encoding format data 1320. Said conversion may be performed by the camera device 131, by the controller 150, or any electronic component arranged in the wireless surgical device 100.
S1009: Converting (e.g., packetizing), e.g., by the multimedia server 1520, the video encoding format data 1320 into a Real-Time Protocol 1330 or a Bluetooth protocol.
S1010: Transmitting, by the transceiver 160, multimedia content (i.e., at least image data derived at least partially from the raw image data 1310) to the first user device 201 in a wireless manner, particularly, the transmitted video content is the video encoding format data 1320, packetized using the Real-Time Protocol 1330 and sent simultaneously and synchronously to the one or more user devices 200 in a wireless manner, once the one or more user devices 200 is/are connected to the local network.
S1011: Receiving, by a receiver 261 of the first user devices 201, the video encoding format data 1320, packetized using the Real-Time Protocol 1330, from the transceiver 160 of the surgical device 100.
S1012: Decoding, by the first user device 201, the video encoding format data 2610 packetized with the Real-Time Protocol into a video encoding format data 2610'.
S1013: Displaying, by the screen device 270, the video encoding format data 2610'.
S1014: Sending, by the transmitter 262, the recording request 210 generated by a HTTP request to the wireless surgical device 100. The transceiver 160 is electrically connected to the multimedia server 1520. The multimedia server 1520 (e.g., the API 1340) is configured to receive the recording request 210. In this way, the captured image in video encoding format data 1320 is stored or recorded on the read-only memory (ROM) (at least during a moment of the surgery). In this way, the user 300 is able to watch again the recorded video (e.g., with a simple interaction to the first user device 201) in real time and on-the-fly. Advantageously, the first user device 201 does not necessarily have to store the recorded video.
S1015: Sending, by the transmitter 262, the configuration request 220 generated by a HTTP request to the wireless surgical device 100. The transceiver 160 is electrically connected to the multimedia server 1520. The multimedia server 1520 (e.g., the API 1340) is configured to receive the configuration request 220. The API 1340 is connected to camera controller (e.g., the image sensor 130). The API 1340 is configured to generate the instruction for changing at 0least one configuration value in real time and on-the-fly of the image contrast, brightness, resolution, and frame rate (at least during a moment of the surgery).

Although only a number of examples of the present electronic device and assembly method thereof have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. Reference signs related to drawings placed in parentheses in a claim are solely for attempting to increase the intelligibility of the claim, and shall not be construed as limiting the scope of the claim.

## Claims

1. A surgical device (100) comprising:
- a light connector (122) configured at least to receive visible light such that the surgical device (100) illuminates an interior portion of an anatomic body (90);
- an optical device (120) including an optical body;
- an image sensor (130) in optical communication with the optical body, the image sensor (130) being configured to acquire a captured image of the interior portion of the anatomic body (90) illuminated by the visible light, wherein the image sensor (130) generates raw image data (1310);
- a circuit board (140);
- a controller (150) attached to the circuit board (140), the controller (150) being electrically connected to the image sensor (130); and
- a transceiver (160) being in electrical connection to the controller (150),
wherein the controller (150) is configured at least to
transmit image data derived at least partially from the raw image data (1310) to one or more user devices (200) in a wireless manner through the transceiver (160).

2. The surgical device of claim 1, wherein it is configured to create a local network,
wherein the controller (150) comprises an identity server (1510) and a multimedia server (1520),
wherein the identity server (1510) and the multimedia server (1520) are arranged within the surgical device (100), the surgical device (100) being a one-single device,
wherein the identity server (1510) is configured to assign dynamic identities to the one or more user devices (200) connected to the local network, and
wherein the multimedia server (1520) is configured at least to transmit the image data derived at least partially from the raw image data (1310) simultaneously and synchronously to the one or more user devices (200) in a wireless manner through the transceiver (160) once the one or more user devices (200) is/are connected to the local network.

3. The surgical device of claim 2, wherein the multimedia server (1520) is configured to receive video encoding format data (1320) based on the raw image data (1310), and to packetize said video encoding format data (1320) into a Real-Time protocol (1330) or a Bluetooth protocol to be transmitted simultaneously and synchronously to the one or more user devices (200) in a wireless manner through the transceiver (160) once the one or more user devices (200) is/are connected to the local network.

4. The surgical device of claims 2-3, wherein the local network operates approximately at 2.4 GHz, and preferably the controller (150) is configured to create the local network.

5. The surgical device of claims 2-4, wherein the identity server (1510) is configured to verify a data request to give access to the one or more user devices (200), preferably, the identity server (1510) is a DHCP server.

6. The surgical device of any preceding claim, wherein the controller (150) is further configured to
∘ convert the raw image data (1310) into a video encoding format data (1320),
∘ packetize, preferably by the multimedia server (1520), the video encoding format data (1320) into a Real-Time protocol (1330) or a Bluetooth protocol, and
∘ transmit at least video encoding format data (1320) packetized using the Real-time protocol (1330) or the Bluetooth protocol to the one or more user devices (200) in a wireless manner through the transceiver (160) once the one or more user devices (200) is/are connected to the local network.

7. The surgical device of claims 3-6, wherein the video encoding format data (1320) is H264, and the Real-Time Protocol (1330) is WebRTC.

8. The surgical device of any preceding claim, wherein it is a wireless surgical device, wherein it further comprises a light-supply device (110) in light connection to the light connector (122), wherein the light-supply device (110) includes a light source to illuminate the interior portion of the anatomic body (90), wherein the surgical device (100) further comprises a camera device (131) that includes the image sensor (130), and wherein both the light-supply device (110) and the camera device (131) do not comprise connecting cables or wires coming out from the surgical device (100).

9. The surgical device of any preceding claim, wherein the optical device (120) comprises an elongated portion (121) defining an optical axis (O), in use, the elongated portion (121) is at least partially introduced to the interior portion of the anatomic body (90), wherein the surgical device further comprises an electronics support (132) arranged substantially perpendicular to the optical axis (O), wherein the image sensor (130) is attached to the electronics support (132), and wherein the circuit board (140) is arranged substantially perpendicular to the electronics support (132),
optionally, the surgical device (100) further comprises a high-bandwidth image data transmission medium (170) electrically connecting the image sensor (130) and the controller (150) to each other for transmitting the raw image data (131) or image data derived therefrom to the controller (150), more optionally, the high-bandwidth image data transmission medium (170) is a flat flexible cable, or a flexible printed circuit board, or a CSI cable.

10. The surgical device of any preceding claim, wherein it further comprises a power source (180) configured to supply electric power to the controller (150), the image sensor (13), and optionally to the light-supply device (110), wherein the power source (180) is optionally a single battery unit, for example, having a voltage of between 1.5 volts and 20 volts.

11. The surgical device of claim 10, wherein it further comprises a housing assembly (190) that is adapted for enclosing the circuit board (140), the controller (150), the transceiver (160), and the power source (180).

12. A wireless surgical system (10) comprising:
- the surgical device (100) according to any preceding claims; and
- the one or more user devices (200),
wherein the one or more user devices (200) are configured to
∘ access the controller (150) of the surgical device (100),
∘ receive the video encoding format data (1320) packetized using the Real-Time protocol (1330),
∘ depacketize the video encoding format data (2610) packetized with the Real-time protocol, and
∘ preferably, display the video encoding format data (2610').

13. The wireless surgical system of claim 12, wherein the one or more user devices (200) is configured to send a recording request (210) to the surgical device (100) such that the controller (150) is capable of recording image data derived at least in part from the raw image data (1310), and/or
wherein the one or more user devices (200) is configured to send a configuration request (220) to the surgical device (100) such that the controller (150) is capable of changing at least one configuration value in real time and on-the-fly such as image contrast, brightness, resolution, and/or frame rate;
optionally, the recording request (210) and/or the configuration request (220) is a HTTP request.

14. A method for operating a wireless surgical system comprising:
- preferably, creating a local network;
- preferably, assigning at least one Internet Protocol (IP) address to the one or more user devices (200) to respond at least to a broadcast query by one or more user devices (200);
- preferably, turning on the light-supply device (110);
- inserting at least a portion of a surgical device (100) into an anatomic body (90);
- illuminating an interior portion of the anatomic body (90);
- acquiring, by a sensor image (130), a captured image of the interior of the anatomic body (90) that includes generating a raw image data (1310);
- preferably, transmitting, by a high-bandwidth image data transmission medium (170), the raw image data (1310) into a controller (150), wherein the controller (150) is an integral part of the surgical device (100);
- preferably, converting encoding the raw image data (1310) into a video encoding format data (1320);
- preferably, converting, by the controller (150), the video encoding format data (1320) into a Real-Time Protocol (1330) or a Bluetooth protocol; and
- transmitting, by a transceiver (160), at least image data derived at least partially from the raw image data (1310) to one or more user devices (200) in a wireless manner, preferably, transmitting the video encoding format data (1320) encoded with the Real-Time Protocol (1330) or the Bluetooth protocol simultaneously and synchronously to one or more user devices (200) in a wireless manner once the one or more user devices (200) is/are connected to the local network.

15. The method of claim 14, wherein it further comprises:
- receiving, by a receiver (261) of the one or more user devices (200), the video encoding format data (1320) packetized with the Real-Time Protocol (1330) from the transceiver (160) of the surgical device (100); and
- depacketizing, by the one or more user devices (200), the video encoding format data (2610) packetized with the Real-Time Protocol into a video encoding format data (2610'),
optionally, it further comprises at least one of the following:
- sending, by the one or more user devices (200), a configuration request (220) generated by a HTTP request;
- sending, by the one or more user devices (200), a recording request (210) generated by a HTTP request; and
- displaying, by the one or more user devices (200), the video encoding format data (2610').
